# EUROPEAN PATENT SPECIFICATION

(11) **EP 2 729 127 B1**
(45) Date of publication and mention of the grant of the patent: **02.05.2018**
(21) Application number: 12746008.7
(22) Date of filing: 03.07.2012
(51) Int. Cl.: A61K 9/127

(54) **METHODS FOR PRODUCING LIPOSOMES**
VERFAHREN ZUR HERSTELLUNG VON LIPOSOMEN
PROCÉDÉ DE PRODUCTION DE LIPOSOMES

(30) Priority: 04.07.2011 DK 201100507
(43) Date of publication of application: 14.05.2014
(73) Proprietor: Statens Serum Institut, 2300 Copenhagen S (DK)
(72) Inventor: ANDREASEN, Lars, Vibe, DK-5000 Odense C (DK); WOOD, Grith, Krøyer, DK-2770 Kastrup (DK); CHRISTENSEN, Dennis, DK-1853 Frederiksberg C (DK)
(74) Representative: Plougmann Vingtoft a/s
(86) International application number: PCT/DK2012/000080
(87) International publication number: WO 2013/004234

(56) References cited:
- WO-A1-2009/003474
- CN-B- 101 338 322
- US-A1- 2004 052 733
- US-A1- 2006 018 828
- ROSENKRANDS IDA ET AL: "Cationic liposomes containing mycobacterial lipids: a new powerful Th1 adjuvant system", INFECTION AND IMMUNITY, AMERICAN SOCIETY FOR MACROBIOLOGY, USA, vol. 73, no. 9, 1 September 2005 (2005-09-01), pages 5817-5826, XP002499002, ISSN: 0019-9567, DOI: 10.1128/IAI.73.9.5817-5826.2005
- RUOZI B ET AL: "Atomic force microscopy and photon correlation spectroscopy: Two techniques for rapid characterization of liposomes", EUROPEAN JOURNAL OF PHARMACEUTICAL SCIENCES, ELSEVIER, AMSTERDAM, NL, vol. 25, no. 1, 1 May 2005 (2005-05-01), pages 81-89, XP027803639, ISSN: 0928-0987 [retrieved on 2005-05-01]
- GERT STORM ET AL: "Liposomes: quo vadis?", PHARMACEUTICAL SCIENCE & TECHNOLOGY TODAY, vol. 1, no. 1, 1 April 1998 (1998-04-01), pages 19-31, XP055056072, ISSN: 1461-5347, DOI: 10.1016/S1461-5347(98)00007-8

## Description

### Field of invention

The present invention relates to liposome manufacturing and formulations. In particular the present invention relates to the manufacturing and formulation of cationic liposomes by a unique combination of lipid powders obtainable by organic solvent-free processes (fx critical fluid technologies) and high shear mixing whereby liposome components are mixed and formulated into stable liposomes without using organic solvents during the process. The liposomes can be used either as an adjuvant for antigenic components or as a drug delivery system in therapeutic and preventive medicines. In particular the invention relates to adjuvants for vaccines in aqueous media for immunization.

### Background of the invention

The term liposome is a broad definition for vesicles composed of lipid bilayers enclosing aqueous compartments. The membrane-forming lipids are amphiphilic and accordingly contain a polar and an apolar region. The polar region typically consists of a phosphate group, an acidic group and/or tertiary or quaternary ammonium salts and can either have a net negative (anionic), neutral or positive (cationic) surface charge at physiological pH, depending on the composition of the lipid head groups. The apolar region typically consists of one or more fatty acid chains with ≥ 8 carbons and/or cholesterol. The lipids constituting the vesicular bilayer membranes are organized such that the apolar hydrocarbon "tails" are oriented toward the center of the bilayer while the polar "heads" orient towards the in- and outside aqueous phase, respectively.

One method to obtain homogeneous liposomes involves dissolving phospholipids in an organic solvent which is then evaporated to dryness leaving a thin lipid film on the inside of the test tube (Bangham et al., 1965). The dry lipid film is then hydrated in an appropriate amount of aqueous phase and the mixture is heated to above the phase transition temperature Tₘ of the lipids/lipid mixture and lipid film is allowed to "swell". The resulting liposomes which typically consist of multilamellar vesicles (MLV's) are dispersed by shaking the test tube. This preparation provides the basis for producing unilamellar vesicles (UV) by methods such as sonication (Papahadjopoulos et al., 1967) or extrusion as described by Cullis et al. in U.S. Pat. No.5,008,050.

Other techniques used to prepare vesicles are reverse-phase evaporation introduced by Szoka and Papahadjopoulos (Szoka and Papahadjopoulos, 1978; U.S. Pat. No. 4,235,871). This technique consists of forming a water-in-oil emulsion of lipids in an organic solvent and an aqueous buffer solution containing a substance to be encapsulated. Removal of the organic solvent under reduced pressure produces a viscous gel. When this gel collapses an aqueous suspension of lipid vesicles is formed.

Preparation of liposomes of phospholipids by the double emulsion method for encapsulating a water soluble compound, e.g. a protein, which does not have strong affinity to the liposome is described by Schneider in US 4,008,801 and US 4,224,179. The emulsion with liposomes containing the compound is prepared by the double emulsion method: A small volume of an aqueous solution with a water soluble compound e.g. a protein antigen and/or an immunostimulator is mixed with a larger volume organic solvent with the phospholipid dissolved. The formed emulsion is mixed with a larger volume aqueous solution and the organic solvents are removed with an airstream.

A method described by Carmona-Ribeiro and Chaimovich (Ribeiro and Chaimovich, 1983) involves injecting an organic e.g. chloroform, methanol, ethanol, solution of the desired lipids into an aqueous buffer where the lipids spontaneously forms liposomes as the solvent evaporates. The ethanol injection method for large scale manufacturing of liposomes has been evaluated in recent years (Wagner 2006, Justo 2010), and this manufacturing method was judged more feasible to large scale production than for example the lipid film method.

Several ways are known to obtain homogeneous lipid powders from organic solvent solutions, such as freeze-drying, spray-drying, solvent evaporation (Mehnert and Mäder, 2001). In these processes the organic solvent in which the lipids are dissolved is removed by the described techniques. The use of organic solvents has a number of disadvantages due to many processes leaving residual solvents in the medicinal product. Furthermore, on upscaling, strict attention is needed with regard to personnel as well as environmental concerns. Also hot or cold homogenization processes have been investigated for manufacturing of lipid powders (Mehnert and Mäder, 2001).

In recent years, lipid powders have alternatively been produced by supercritical fluid methods which were developed for producing stabilized suspensions of water insoluble drugs (Badens et al, 2001, Cape et al, 2008). The supercritical fluid process applies a compressed gas, e.g. CO₂, in its supercritical state as solvent in some processes or as anti-solvent in other processes (Pasquali et al, 2008). This can help to produce submicron particles by precipitating the water in-soluble compound by rapid expansion from a supercritical solution in which the compound is liquefied or dissolved/partly dissolved or by applying the anti-solvent approach by spraying a solution of the compound into a compressed gas, liquid or supercritical fluid. The process is well known for producing phospholipid vesicles (WO9714407, US 5,554,382, WO2010004287). Most often, organic solvents or added co-solvents are applied.

Liposomes for encapsulation manufactured directly from scCO₂ solution have been investigated applying ethanol as co-solvent for liposomes made up of phospholipids and cholesterol (Frederiksen et al, 1997).

Techniques to manufacture liposome entrapped/encapsulated substances, such as macromolecules, DNA/RNA, enzymes, proteins, peptides and hormones, have been investigated. One example of a liposome entrapment technique is the dehydration-rehydration procedure (Gregoriadis 1987, 1993, 1999, Kirby et al. 1984, Zadi, 2000). In this method, liposomes are preformed by e.g. the lipid film hydration method described above, in which the applied organic solvents are removed prior to the dehydration-rehydration process. The lipid films are hydrated by use of water/buffer to form "empty" liposomes or, alternatively, by use of an aqueous solution holding the substance to be encapsulated. The hydration procedure can be done by known techniques e.g. by heating the aqueous solution till above the phase transition temperature Tₘ of the lipid components and whirl-mixing at intervals. Subsequently, the liposome preparation is freeze-dried, and afterwards rehydrated under controlled conditions above the gel-liquid crystal transition temperature of the lipids, first with a small amount of water or solution holding the substance, thereafter diluted to the finale volume. The resulting dehydration-rehydration vesicles (DRV) can be processed by microfluidization to appropriate size and the remaining non-entrapped substance removed by chromatography, centrifugation steps etc. Size reduction after entrapment, however, tends to lead to lower entrapment load and efficiency.

All of these methods however include the use of organic solvents/co-solvents. With many organic solvents being known carcinogens and/or having damaging effects on the central nervous system it is important to reduce the content of residual organic solvents in medicinal products for human use and to document that the trace amounts that are left in the medicinal product do not exceed the tolerable pharmacopeia levels. Furthermore the use of organic solvents in production results in large amounts of these compounds for disposal. There are therefore many rules and regulations to be kept when using organic solvents which makes the production process more complicated. With the protection gear and equipment used today, it is possible to minimize the exposure to personnel and maintain a working environment of high standard. Therefore the authorities today are much more focused on the exposure to the outer environment. When volatile components such as organic solvents are removed from the work environment by suction to the outer environment, then these solvents can contribute to the formation of photochemical pollution in the air such as ozone or smog. Therefore new governmental initiatives include a guide for limitation of air pollution from the industry. This guideline includes keeping a very accurate balance of volatile organic components, so everything that comes in and goes out needs to be registered. This makes it very time consuming and expensive to work with organic solvents in the modern industry. The use of a production method free for organic solvent in the present invention is therefore highly favorable.

The application of liposomes as vaccine delivery systems and adjuvants has been investigated extensively over the last few decades. Cationic liposomes are the most effective liposomal delivery systems for vaccine antigens and are more potent immunopotentiators than other liposome systems. However, although there is some evidence that cationic liposomes themselves can improve the immune response against co-administered vaccine antigens, their main functions are to protect the antigens from clearance in the body and deliver the antigens to professional antigen-presenting cells (Christensen et al. 2007). Recent studies suggest that the cationic charge is an important factor for the retention of the vaccine at the site of injection, enabling prolonged antigen presentation to and immunostimulation of the innate immune cells (Henriksen-Lacey et al. 2010). In addition, cationic liposomes can be used to introduce immunostimulators to enhance and modulate the immune response in a desirable direction and, thereby, represent an efficient tool when designing tailor-made adjuvants for specific disease targets (Christensen et al. 2007). The cationic surfactant components constituting the liposomes might include surfactants like dimethyldioctadecylammonium (DDA), 3-[N-(N',N'-dimethylaminoethane)-carbamoyl] cholesterol (DC-Chol), 1,2-acyl-3- trimethylammonium-propane (DxTAP), N-[1-(2,3-acyloxy)propyl]-N,N,N-trimethylammonium (DxTMA), acyloxy(ethyl-2-acyl-3-hydroxyethyl) imidazolinium (DxTIM), 1,2-acyl-glycero-3-ethylphosphocoline (DxEPC), 3-tetradecylamino-tert-butyl-N-tetradecylpropionamidine (diC14-amidine), N-palmitoyl-D-erythrospingosyl-1-O-carbamoyl spermine (CCS) and dimethyldiacylammonium (DDxA) where x represents acyl chains with varying chain lengths such as: lauroyl (C12), myristoyl (C14), palmitoyl (C16), stearoyl (C18), arachidoyl (C20) and behenoyl (C22).

The immunogenicity of these liposomes can be potentiated by inclusion of immunostimulating ligands (a.k.a. immunopotentiators) for the so-called pattern recognition receptors (PRRs) recognizing conserved molecular structures known as pathogen-associated molecular patterns (PAMPs). The ability of the PAMP to modulate the innate immune response, and thereby the ensuing adaptive response, can with advantage be exploited for use in vaccines. The PRRs include C-type lectin receptors, nucleotide-binding oligomerization domain (NOD) proteins, and the ever-growing toll-like receptor (TLR) family. The PAMPs vary among the pathogens and include molecules such as cord factor (TDM), flagellin, lipopolysaccharide (LPS), peptidoglycans, and several nucleic acid variants, such as cytosine:phosphate:guanine (CpG) oligodeoxynucleotides and double-stranded ribonucleic acids (dsRNAs). Many immunostimulators inspired by the PAMPS have been developed over the years. These include, trehalose dibehenate (TDB), synthetic monomycolyl glycerol (MMG), monophosphoryl lipid A (MPL), polyinosinic acid:polycytidylic acid (poly(I:C)). The combination of liposomes with these PAMPs/immunostimulators is an attractive approach to develop a vaccine adjuvant, since the liposomes not only deliver the vaccine antigens, but also the PAMPs/immunostimulators to the antigen presenting cells, thereby potentiating the immune response.

A large number of adjuvants that induce a cell-mediated immune response have been suggested but in general without any being ideal in all respects.

One particular effective type of adjuvant that promotes a cell-mediated immune response is the class of quaternary ammonium compounds, such as dimethyldioctadecylammonium (DDA). DDA is a synthetic amphiphile comprising a hydrophilic positively charged dimethylammounium head group and two long hydrophobic alkyl chains. In an aqueous environment DDA molecules self-assemble to form vesicular bilayers similar to liposomes made from natural phospholipids. Combinations of DDA and other immunopotentiating agents are well-known from the literature.

Unfortunately, suspensions of amphiphilic quaternary ammonium compounds such as DDA alone are physically unstable and prolonged storage at 4 °C is not possible without the occurrence of aggregation and precipitation. As precipitation will prevent the clinical use of the formulation, the lack of stability of DDA formulations has so far been a major obstacle for any application in humans.

Several ways to stabilize suspensions of amphiphilic quaternary ammonium compounds have been envisioned. These include the stabilization of the bilayer with e.g. polyethyleneglycol (PEG), glycolipids (such as TDB) and polymers. WO2006002642 discloses liposome formulations that are physically stable. The document relates to steric stabilization of cationic liposomes by incorporating glycolipids into the lipid bilayer as well as increased hydration of the lipid membrane. The stabilized liposomes can be used either as an adjuvant for antigenic components or as a drug delivery system. In particular the document relates to vaccines with adjuvants in aqueous media for immunization, where the final product is stable but not free of organic solvents.

In addition WO2006002642 discloses the addition of other immunopotentiators than the glycolipids to the vesicle formulation e.g. poly(I:C) and CpG, though it does not suggest how these further combinations may be made into a stable formulation as the incorporation of these immunopotentiators makes the formulation unstable. Further addition of immunopotentiators and/or antigens into stable formulations can however cause destabilization. In particular polyanions like poly(I:C) are difficult to formulate into cationic liposomes and liposome adjuvants due to the fact that the binding of highly charged polyelectrolytes to oppositely charged liposomes promotes the lipid bilayer membrane destabilization per se. Charge neutralization and concomitant reduction of the outer surface area leads to a gradient in lateral pressure in the direction of the bilayer normal. This generates a bending moment, which in turn contributes to destabilizing the liposomes resulting in the formation of larger aggregates over time, broad size distribution and structural heterogeneity resulting in precipitation of the liposomes.

There are a few methods for incorporating/complexing oligonucleotides, peptides or proteins in/with liposomes described in literature, where the liposomes have been prepared prior to incorporation/complexing using organic solvent: In one method oligonucleotides, peptides or proteins present in the aqueous phase are partially entrapped by freeze drying followed by rehydration of the lyophilized liposomes (Kirby and Gregoriadis, 1984).

Alternatively the peptides or proteins are incorporated using the freeze and thaw technique described by Pick (Pick, 1981) and by Bally et al. in U.S. Pat. No. 4,975,282. In this technique preformed vesicles produced by the above mentioned process are mixed with the peptides or proteins and repeatedly snap frozen in liquid nitrogen and warmed to temperatures above the phase transition temperature Tₘ of the relevant lipids. As this method applies pre-formed liposomes, the above mentioned methods for producing such also apply here, e.g. uses of organic solvent and difficulties in upscaling the process, such as snap-freezing larger batches.

Recent example for adsorption of high concentrations of hydrophilic anionic compounds e.g. polyanions like poly(I:C) to the liposomes of cationic lipids of quarternary ammonium compounds using organic solvents and the double emulsion method is described in (Nordly et al. 2011).

Rosenkrands et al. (Infection and Immunity, vol. 73, no. 9: 5817-5826 (2005)) discloses dissolving liposome-forming compounds in chloroform, followed by evaporation of the solvent. Preparation of the liposomes included mixing by sonication.

Ruozi et al. (Eur. J. Pharm. Sci., vol. 25, no. 1: 81-89 (2005)) discloses a method of producing liposomes using chloroform and including the use of sonication.

WO 2009/003474 discloses preparation of liposomes comprising DDA, glycolipid MMG as adjuvant, TDB and Ag85B-ESAT-6 antigen, but without the use of e.g. high-shear mixing.

Storm and Crommelin (Pharm. Sci. & Techn. Today, vol. 1, no. 1: 19-31 (1988)) provide a review of liposomes as a delivery system for therapeutic agents.

US 2006/0018828 A1 relates to a radiographic contrast medium containing liposome vesicles prepared using a supercritical carbon dioxide method, but does not disclose high shear mixing.

US 2004/0052733 A1 relates to pharmaceutical compositions for inhalation comprising active particles and cholesterol particles. It mentions supercritical fluid methods as one option that may be suitable for making fine cholesterol particles, but states that preferably, the cholesterol is not present in the form of liposomes or as a component in liposomes.

The present invention discloses a method for the manufacturing and formulation of cationic liposomes by an organic solvent-free method from lipid powders obtained from organic solvent-free methods. The present invention further discloses a method to formulate stable cationic liposomes with added polyanioinic immunopotentiators.

### Summary of the invention

The present invention discloses a new method for the formation of liposomes without using organic solvents by rehydration of lipid powders obtainable without the use of organic solvents or co-solvents by using high shear mixing. This invention use the supercritical fluid technology to create a homogeneous lipid powder but the lipid powder can be produced by other known techniques. Components of the liposomes include but are not limited to cationic lipids, immunostimulators/immunopotentiators and macromolecules as components for the liposome formation.

This invention discloses a method for the formulation of stable liposomes solitary or complexing high concentrations of macromolecules such as proteins, DNA and RNA having opposite charge of the liposomes by high shear mixing where aggregation is avoided due to the disclosed formulation method.

The present invention also discloses the use of these stabilized liposomes complexed with poly-anionic immunostimulants as vaccine adjuvants.

### Detailed disclosure of the invention

The present invention discloses a method of producing liposomes using high shear mixing of a lipid powder in an aqueous solution according to claim 1.

The aqueous solution can be pure water or an aqueous solution containing buffers, salt, sugars, macromolecules, acid, bases, amino acids, peptides, proteins, etc. The pH of the solution can be between pH 1-14, preferably pH 5-9 and most preferred pH 6-8.

In a preferred embodiment of the invention the lipid powder is produced by a supercritical fluid process.

The preferred lipid powder is a homogeneous mixture where one lipid component is quaternary ammonium compounds e.g. where the quarternary ammonium compound is dimethyldioctadecylammonium (DDA), or dimethyldioctadecenylammonium (DODA), or 1,2-dioleoyl-3-trimethylammonium propane (DOTAP), 1,2-dimyristoyl-3-trimethylammonium-propane, 1,2-dipalmitoyl-3-trimethylammonium-propane, 1,2-distearoyl-3-trimethylammonium-propane and dioleoyl-3-dimethylammonium propane (DODAP), N-[1-(2,3-dioleyloxy)propyl]-N,N,N-trimethylammonium (DOTMA), octadecenoyloxy(ethyl-2-heptadecenyl-3-hydroxyethyl) imidazolinium (DOTIM) 1,2-dioleyl-sn-glycero-3-ethylphosphocoline (DOEPC) and 3-tetradecylamino-tert-butyl-N-tetradecylpropion-amidine (diC14-amidine).

The liposomes of the invention can further comprise a neutral lipid such as a phospholipid, a stabilizing glycolipid such as alpha,alpha'-trehalose 6,6'-dibehenate (TDB) or monomycolyl glycerol (MMG) or syntetic analogs hereof, an immunepotentiator such as C-type lectin receptors, nucleotide-binding oligomerization domain (NOD) proteins and the toll-like receptor (TLR) family, such as cord factor (TDM) or the synthetic analogue TDB, flagellin, lipopolysaccharide, peptidoglycans or nucleic acid variants, such as cytosine:phosphate:guanine (CpG) oligodeoxynucleotides and double-stranded ribonucleic acids (dsRNAs) like polyinosinic acid:polycytidylic acid (poly(I:C)).

In a further embodiment the produced liposomes are complexed with macromolecules such as oligonucleotides, peptides, proteins, carbohydrates and/or lipids applying a high shear mixing procedure and controlled addition of the solution containing the substance to be complexed with the liposomes.

According to the invention the high shear mixing based on the rotor-stator principle, e.g. where the rotational speed of the rotor is at least 3,400 rpm. Other high-shear mixing techniques include homogenizing and extrusion.

The liposomes produced by the described method can be used for the manufacture of an adjuvant, a delivery system, a pharmaceutical or a cosmeceuticals.

All steps in the present invention are easy to handle under inert atmosphere in case oxidation sensitive components are applied, like phospholipids, unsaturated fatty acids or application of active pharmaceutical ingredients, which are sensitive towards oxidation, deamidation etc.

The present invention also discloses a liposome product stabilized for use as a vaccine adjuvant where the vaccine can be against any disease e.g. influenza, streptococcus, tuberculosis, diabetes, cancer, malaria, chlamydia etc.

Lipid powder or liposomes manufactured or stabilized by the methods in the present invention can be used pharmaceutically either for injection, in delivery systems, topical application, intradermal application, intranasal application, pulmonary application or in other formulations such as but not limited to cosmetics.

### Definitions

"Lipid powders" are defined as a dry, particulate material constituting a mixture of the lipids of the lipid powder formulation. It is desirable for the further use of the lipid powders for liposome manufacturing that the lipid powder is a homogenous mixture of the lipids of the formulation.

"Liposomes" are defined as closed vesicle structures made up of one or more lipid bilayers surrounding an aqueous core. Each lipid bilayer is composed of two lipid monolayers, each of which has a hydrophobic "tail" region and a hydrophilic polar "head" region. In the lipid bilayer, the hydrophobic "tails" of the lipid monolayers orient toward the inside of the bilayer, while the hydrophilic "heads" orient toward the outside of the bilayer. Liposomes can have a variety of physicochemical properties such as size, lipid composition, surface charge, fluidity and number of bilayer membranes. According to the number of lipid bilayers liposomes can be categorized as unilamellar vesicles (UV) or small unilamellar vesicles (SUV) comprising a single lipid bilayer or multilamellar vesicles (MLV) comprising two or more concentric bilayers each separated from the next by a layer of water. Water soluble compounds are entrapped within the aqueous phases/core of the liposomes as opposed to lipophilic compounds which are trapped in the core/center of the lipid bilayer membranes.

The term "cationic lipid" is intended to include any amphiphilic lipid, including natural as well as synthetic lipids and lipid analogs, having hydrophobic and polar head group moieties, a net positive charge at physiologically acceptable pH, and which can form bilayer vesicles or micelles in water.

One particular preferred type of cationic lipids used in this invention is quaternary ammonium compounds having the general formula NR¹R²R³R⁴-X wherein R¹ and R² independently each is a short chain alkyl group containing from 1 to 3 carbon atoms, R³ is independently hydrogen or a methyl or an alkyl group containing from 12 to 20 carbon atoms, preferably 14 to 18 carbon atoms, and R⁴ is independently a hydrocarbon group containing from 12 to 20 carbon atoms, preferably from 14 to 18 carbon atoms. X is a pharmaceutical acceptable anion, which itself is nontoxic. Examples of such anions are halide anions, e.g. chloride, bromide and iodine. Inorganic anions such as sulfate and phosphate or organic anions derived from simple organic acids such as acetic acid may also be used. The R¹ and R² groups can be methyl, ethyl, propyl and isopropyl, whereas R³ can be hydrogen, methyl or dodecyl, tridecyl, tetradecyl, pentadecyl, hexadecyl, heptadecyl, octadecyl nonadecyl and eicocyl groups and R⁴ can be dodecyl, tridecyl, tetradecyl, pentadecyl, hexadecyl, heptadecyl, octadecyl nonadecyl and eicocyl groups. However, also other C₁₂-C₂₀ hydrocarbon groups are possible because even though the R³ and R⁴ groups preferably are saturated with no branched side chains they may in minor degree be branched having e.g. methyl and ethyl side chains. R³ and R⁴ may also have a minor degree of unsaturation, e.g. containing 1 - 3 double bonds each, but preferably they are saturated alkyl groups.
The cationic lipid is most preferably dimethyldioctadecylammonium bromide or chloride (DDA-B or DDA-C) or the sulfate, phosphate or acetate salt hereof (DDA-X), or dimethyldioctadecenylammonium bromide or chloride (DODA-B or DODA-C) or the sulfate, phosphate or acetate compound hereof (DODA-X).
Other preferred cationic lipid compounds are 1,2-dioleoyl-3-trimethylammonium propane (DOTAP), 1, 2-dimyristoyl-3-trimethylammonium-propane, 1,2-dipalmitoyl-3-trimethylammonium-propane, 1,2-distearoyl-3-trimethylammonium-propane and dioleoyl-3-dimethylammonium propane (DODAP), N-[1-(2,3-dioleyloxy)propyl]-N,N,N-trimethylammonium (DOTMA), octadecenoyloxy(ethyl-2-heptadecenyl-3-hydroxyethyl) imidazolinium (DOTIM) 1,2-dioleyl-sn-glycero-3-ethylphosphocoline (DOEPC) and 3-tetradecylamino-tert-butyl-N-tetradecylpropion-amidine (diC14-amidine).

An Alkyl chain refers to an aliphatic hydrocarbon chain which may be straight or branched. The chain can be saturated or it may contain one or more double bounds e.g. being unsaturated.

An acyl chain refers to an alkyl-OC(O) group, wherein the alkyl group is as previously described.

Fatty acid chain refers to a branched or unbranched saturated or unsaturated hydrocarbon chain of alkyl or acyl groups.

The term pharmaceutically acceptable refers to a substance which does not interfere with the effectiveness or the biological activity of the active ingredients and which is not toxic to the host or the patient.

Phase transition temperature or Tₘ is for the present invention the temperature at which the liposomal bilayer physically changes from a lower-temperature gel-phase, characterized by ordered fatty acid chains (a solid-ordered phase) to a high-temperature fluid-phase in which the fatty acid chains have a high degree of conformational disorder (a liquid-disordered phase), as measured by differential scanning calorimetry (DSC).

The cationic liposomes are stabilized by incorporating glycolipids into the liposome membranes. By incorporating is meant procedures to imbed a molecule's hydrophobic region and hydrophilic region in a corresponding hydrophobic and hydrophilic region or moiety of a membrane, micelle, liposome or bilayer. Procedures for incorporating glycolipids in liposomes can be "the thin film method", "the reverse-phase evaporation method" and "the organic solution injection method" and future - at the present time unknown methods having the same effect of incorporating glycolipids into the liposome membranes. All the present known methods are mentioned in the background of invention chapter.

A glycolipid is defined as any compound containing one or more monosaccharide or glycerol residues bound by a glycosidic linkage to a hydrophobic moiety such as a long chain fatty acid, acylglycerol, a sphingoid, a ceramide or a prenyl phosphate. The glycolipids of this invention can be of synthetic, plant or microbial origin e.g. from mycobacteria.

One class of glycolipids used in this invention is acylated (or alkylated) glycosides, which consists of one or two sugars residues esterified to one, two or even three fatty acids. The fatty acids can be either straight chain including saturated fatty acids e.g. myristic acid C14:0, pentadecanoic acid C:15, palmitic acid C16:0, heptadecanoic acid C17:0, steric acid C18:0, nonadecanoic acid C:19, arachidic acid, C:20, heneicosanoic C21:0, behenic acid C:22 and unsaturated fatty acids e.g. oleic acid C18:1n-9 linoleic acid 18:2n-6, or complex branched fatty acids such as mycolic acid, methoxymycolic acids, ketomycolic acids, epoxymycolic acids and corynomycolic acid. The sugar residues can be either simple monosaccharides e.g. glucose and fructose or disaccharides comprising two covalently linked monosaccharides e.g. sucrose consisting of glucose and fructose and trehalose in which two glucose units are joined by a glycosidic linkage. One type of glycolipids used in this invention is cell wall glycolipids isolated from mycobacterium, which consists of a disaccharide esterified to one, two, or three either normal palmitic acid, C16:0; oleic acid, C18:1n-9; linoleic acid, 18:2n-6 or complex hydroxy, branched-chain fatty acids i.e. mycolic acid residues ranging in length from 60 to 90 carbon atoms. Other bacterial glycolipids used in this invention have shorter fatty acid chains e.g. corynomycolic (22-36 carbons) or nocardomycolic (44-60 carbons) acids isolated from Corynobacterium, Nocardia. A preferred mycobacterial glycolipid is alpha,alpha'-trehalose 6,6'-dimycolate (TDM) often referred to as cord factor, which is one of the most important immunomodulatory components of the mycobacterial cell wall. In a particular preferred embodiment the glycolipid consists of the disaccharide alpha,alpha'-trehalose esterified to two docosanoic fatty acids (behenic acid) e.g. alpha,alpha'-trehalose 6,6'- dibehenate (TDB), which is a pure synthetic analog to TDM. Another preferred mycobacterial glycolipid is monomycolyl glycerol or preferably the synthetic analog 3-hydroxy-2-tetradecyl-octadecanoic acid-2,3-dihydroxypropyl ester or closely related compounds with shorter or longer acyl backbones.

Other classes of glycolipids used in this invention include but are not limited to: Glycolipids based on glycerol: These lipids consist of a mono- or oligosaccharide moiety linked glycosidically to the hydroxyl group of glycerol which may be acylated (or alkylated) with one or two fatty acids. Furthermore, these glycolipids may be uncharged and, therefore often called neutral glycoglycerolipids, or may contain a sulfate or a phosphate group. A preferred glycolipid of this class is monomycolyl glycerol (MMG) (Andersen et al 2009a, Andersen et al 2009b) or syntetic analogs hereof, such as 3-hydroxy-2-tetradecyl-octadecanoic acid-2,3-dihydroxypropyl ester or closely related compounds with shorter or longer acyl backbones (Andersen et al 2009b, Nordly et al 2011)

Glycolipids based on ceramides: Glycosphingolipids have according to the structure of the carbohydrate moiety been divided into neutral glycosphingolipids containing an unsubstituted glycosyl group and acidic glycosphingolipids containing a glycosyl group with an acidic carboxyl, sulphate, or a phosphate group.

Lipopolysaccharides (LPS): These complex compounds are the endotoxic antigens found in the cell walls of Gram-negative bacteria (S-lipopolysaccharides). The lipid part (Lipid A) forms a complex with a polysaccharide through a glycosidic linkage. Lipid A consists of a backbone of b-1,6-glucosaminyl-glucosamine with two phosphoester groups in the 1-position of glucosamine I and in the 4-position of glucosamine II. The 3-position of glucosamine II forms the acid-labile glycosidic linkage to the long-chain polysaccharide. The other groups are substituted (in Escherichia) with hydroxylated fatty acids as hydroxymyristate (two ester-linked and two amide-linked) and normal fatty acids (laurate). A particular preferred lipopolysaccharide of this invention is monophosphoryl derivatives of lipid A (MPL), which are non toxic and have excellent adjuvant properties.

Glycosides of sterols: This family consists of one carbohydrate unit linked to the hydroxyl group of one sterol molecule. The sterol moiety was determined to be composed of various sterols: cholesterol, campesterol, stigmasterol, sitosterol, brassicasterol and dihydrositosterol. The sugar moiety is composed of glucose, xylose and even arabinose.

Glycosides of fatty acids or alcohols: A great number of simple glycolipids are found in bacteria, yeasts and in lower organisms (sponges). These compounds are composed of a glycosyl moiety (one or several units) linked to one hydroxyl group of a fatty alcohol or a hydroxy fatty acid or to one carboxyl group of a fatty acid (ester linkage). These compounds frequently possess interesting physical or biological properties. Some of them are industrially produced for their detergent properties (alkyl glycosides).

Moreover, glycolipids e.g. TDB and MMG have immunostimulatory properties themselves and can act in a synergistic way with the quaternary ammonium compounds to enhance the immune response. Furthermore, macromolecules e.g. oligonucleotide, peptide or protein antigens can be entrapped within the aqueous phase of both unilamellar and multilamellar liposomes.

By an immunepotentiator is meant any component which increases the effect of or acts synergistically with the vaccine delivery system and antigens to obtain an immune response. This potentiation could be done non-specifically or specifically through pattern recognition receptors including but not limited to C-type lectin receptors, nucleotide-binding oligomerization domain (NOD) proteins and the toll-like receptors (TLRs)

Complexing of substances includes, but is not limited to, non-covalent binding, e.g. electrostatic interaction, hydrophobic attraction of macromolecules to liposomes of same or opposite charge.

Macromolecules: A macromolecule is defined as a very large molecule, such as a protein, consisting of many smaller structural units linked together; also molecules such as DNA and RNA which are very large and complex are defined as macromolecules. The term is applied to the four conventional biopolymers (nucleic acids, proteins, carbohydrates, and lipids), as well as non-polymeric molecules with large molecular mass. The use of macromolecules in the present invention is including but not limited to oligonucleotides, peptides, proteins, carbohydrates and lipids.

The present invention discloses the homogenous mixing of liposomal lipid components, such as cationic surfactants like DDA and glycolipids like TDB, before hydration and after hydration the potential for subsequent complexing with other macromolecules. Mixing of the lipid components with other macromolecules (e.g. oligonucleotides) can also be done before hydration and have the high shear mixing do the complexing. Complexes between cationic lipids and in particular negatively charged macromolecules (e.g. oligonucleotides) are generally thermodynamically unstable resulting in the formation of larger aggregates over time, a broad size distribution and structural heterogeneity. The binding of highly charged polyelectrolytes to oppositely charged liposomes promotes membrane destabilization per se. Charge neutralization and concomitant reduction of the outer surface area leads to a gradient in lateral pressure in the direction of the bilayer normal. This generates a bending moment, which in turn contributes to the destabilisation of the liposomes.

### Producing the lipid powder

The lipid powder which is a dry, particulate material has to be a homogeneous mixture of the lipids of the formulation. Less homogeneous lipid powders will lead to less stable and less homogeneous liposome formulations. There are several known ways of obtaining homogeneous powders, such as freeze-drying, spray-drying, solvent evaporation all being processes in which an organic solvent is removed from a solution of the lipids in organic solvent. The use of organic solvents has a number of disadvantages due to many processes leaving residual solvents in the medicinal product. Further on up-scaling, focus is on personnel as well as environmental issues.

The present invention discloses a method to avoid the use of organic solvents during the entire liposome manufacturing process from lipid raw materials to formulated cationic liposomes or formulated cationic liposomes with added polyanionic immunopotentiators. The key is to apply homogeneous lipid powders obtainable without the use of organic solvents. A homogenous powder can be obtained by solubilising, liquefying or melting the ingoing starting materials into a homogenous blend, which upon solidifying from either the supercritical fluid or from cooling of the obtained melt result in a lipid powder or a solid lipid powder that can be grinded to powder. One such process is supercritical fluids, for example supercritical CO₂. The lipids are liquefied/dispersed/solubilised in scCO₂ which ensures a homogeneous mixing of the lipids. The resulting dry lipid powder is obtainable by rapid expansion of the scCO₂ holding the lipid mixture. In this way, a fine particulate lipid powder is obtained.

Other types of achieving a homogenous powder in this invention include but are not limited to simple melting of the compounds. This can be carried out with or without pressure, with or without active cooling and with or without mechanical agitation, both during or following the melting process e.g. stirring or granulation.

Preferably the lipid powder is produced by using the supercritical fluid technology to create a homogeneous lipid powder, but any lipid powder can be used as the basis material for liposome production.

### Producing the liposomes

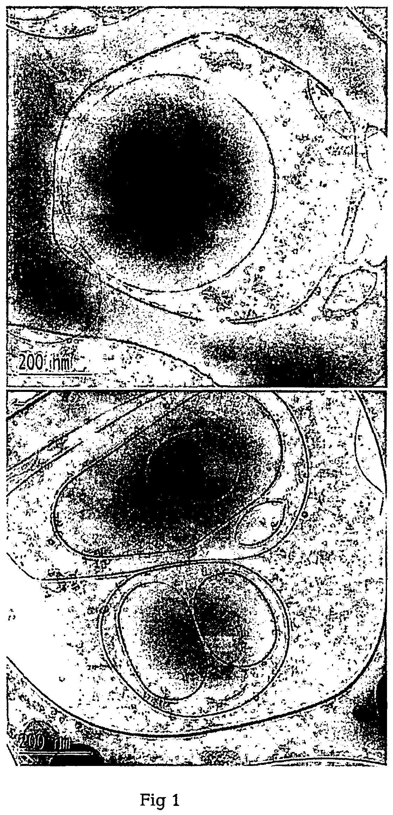

According to the present invention liposomes are formulated from lipid powder without the use of organic solvents in a simple way, using high shear mixing during the lipid powder hydration step (Figure 1). This high shear mixing method can be used to complex oppositely charged macromolecules, such as oligonucleotides, peptides or proteins with the liposomes, obtaining stable liposome-macromolecule complexes.

The result of high shear mixing is the mixing of at least two substances that: do not dissolve in each other, hardly dissolve in each other, or do not chemically react with each other. One such example of two immiscible substances is the hydration of lipids into an aqueous buffer solution to form liposomes. During high shear mixing one substance is distributed throughout another substance.

The present invention uses high shear mixing based on the rotor-stator principle. Here, the rotor is moved with a high circumferential speed. The rotation produces suction, which pulls the medium into the rotor and then pushes it to the outside with help from the stator's teeth. This ensures a thorough mixing of the liposome formulation and any added immunopotentiators or other substances. Stable liposomes and liposome-macromolecule complexes are formed with rotational speeds from 3400 to 40000 rpm; rotational speeds are today limited to about 40000 rpm but there seems no upper limit for making the stable liposome solution. Temperatures are kept below boiling point and above 5°C during the manufacturing.

Other types of high shear mixing include homogenizing, ultrasound and extrusion. Homogenizing is carried out by intensive blending of the liposomes or liposome-macromolecule complexes. Ultrasound is used to mix and downsize multilamellar (micron size) vesicles manufactured by heating and agitation into small (nano size) unilamellar vesicles. Extrusion is used to mix and downsize multilamellar vesicles manufactured as above where liposomes of defined size and homogeneity are prepared by sequential extrusion through polycarbonate membranes.

The methods presented in the present invention are used to manufacture liposome formulations and liposome-macromolecular complexes including incorporation or encapsulation of macromolecules and immunomodulators.

Using the present method, one can form stable liposome formulations and in particular stable formulations of liposome-macromolecule complexes. In the present method, one can associate practically all substances which have an electrostatic affinity for the compounds of the liposomal membrane. Electrostatic affinity is the affinity between two or more compounds created when numerous opposite charged atoms form ionic bonds. The manufactured liposomes are cationic with a highly positive ζ-potential. This results in a high degree of adsorption of substances including but not limited to e.g. macromolecules with a pl below 7.4 at physiological pH, since they are negatively charged and possess an electrostatic affinity towards the positively charged liposomes.

The liposomes and liposome-macromolecule complexes formed by methods presented in the present invention are very stable over time. By stability of a pharmaceutical formulation the capacity of the formulation to remain within defined limits during the shelf life of the product is meant. Liposomal dispersions exhibit chemical as well as physical stability characteristics.

Chemical stability is related to chemical degradation whereas physical stability relates to the colloidal stability of the system.

The physical stability of liposomal dispersions is determined by the inter-vesicular interactions, which depends on the balance between attractive and repulsive forces. Colloidal systems are stabilized by repulsive forces i.e. electrostatic repulsion and steric repulsion due to differences in chemical potential between water in the bulk and in the interaction region.

The invention also presents the use of these liposomes as an adjuvant e.g. for use in vaccine compositions. In particular the invention relates to vaccines with adjuvants in aqueous media for immunization, where the final product is stable.

An adjuvant is an agent that may increase the efficacy or potency of other compounds but has few or no pharmacological effect by itself. An adjuvant can also work as a stabilizing agent or a delivery system. Adjuvants are widely used in human therapies such as a broad range of vaccines, combination therapy (where more than one medication is used), cancer therapies e.g. chemotherapy, hormone therapy, radiation therapy, immunotherapy and targeted therapy mainly small molecules and monoclonal antibodies. In the cosmetic industry adjuvants are mainly used in cosmeceuticals within the area of skin cancer, photoaging and in wound healing but can be used in other applications as well. Adjuvants are also used in agriculture both in animal health and in agricultural sprays e.g. to enhance the performance of pesticides, fungicides, herbicides, feeding stimulants etc.

### Figure legends

**Fig. 1** Cryo-TEM picture of CAF01 liposomes manufactured by high-shear mixing. The formulation contains small unilamillar vesicles, either as SUVs or as multi-vesicular UV's.
**Fig. 2** DSC analysis of DDA and TDB as single component raw materials prior to the super critical fluid method. Microcalorimeter from TA instruments was used for the measurements.
**Fig. 3** DSC analysis of DDA/TDB lipid powder (DDA:TDB ratio 5:1 w/w) produced using the super critical fluid method (RESS). Microcalorimeter from TA instruments was used for the measurements. The effect of stirring and no stirring during super critical fluid method is compared for processes without use of organic solvents.
**Fig 4****.** DSC analysis of DDA/TDB lipid powder (DDA:TDB ratio 5:1 w/w) manufactured by supercritical CO₂ method in standard autoclave. The peaks of the lipid powder are clearly different from those of the DDA and TDB raw materials shown in Figure 2. Microcalorimeter from TA instruments was used for the measurements
**Fig 5****.** DSC thermograph of DDA and CAF01 (DDA/TDB) liposomes rehydrated using high shear mixing. Scans from 30-55°C with 30°C/h, operating pressure 35 psi. VP-DSC Microcalorimeter (Microcal, Northhampton, MA).
**Fig 6****.** Size distribution of CAF01 (DDA/TDB) liposomes rehydrated using high shear mixing. Size distribution was measured using a Malvern Zetasizer-Nano from Malvern Instruments Ltd., UK.
**Fig 7**.
   A) Time development of average particle size of CAF01 (DDA/TDB) liposomes prepared by high-shear mixing at 1 6000 rpm (-●-), 12000 rpm (-■-) and 24000 rpm (-▲-). The liposomes were dispersed in 10mM Tris buffer adjusted to pH 7.4. CAF01 liposomes prepared at 6000 rpm aggregated to a degree rendering it impossible to make further measurements by PCS after 71 days.
   B) Release of IFN-gamma from blood lymphocytes isolated from C57BI/6j mice immunized with 2 µg of Ag85B-ESAT-6 in CAF01 (DDA/TDB) prepared by either the ultrasound or the high shear method. Blood lymphocytes were isolated 1 week after the third immunization and restimulated in vitro with 0.05, 0.5 and 5 µg/ml of Ag85B-ESAT-6.
**Fig. 8****.**
   A) Release of IFN-gamma from blood lymphocytes isolated from C57BI/6j mice immunized with 5 µg of ovalbumin in CAF05 (DDA/TDB/poly (I:C)) prepared by the high shear mixing method at 6000, 12000 and 24000 rpm. Splenocytes were isolated 3 weeks after the third immunization and restimulated in vitro with 5 µg/ml of ovalbumin.
   B) % SIINFEKL-positive CD8+ T-cells after three immunizations with ovalbumin in CAF05 (DDA/TDB/poly (I:C)) prepared by the high shear mixing method at 6000, 12000 and 24000 rpm. Splenocytes were isolated 3 weeks after the third immunization and restimulated in vitro with 5 µg/ml of SIINFEKL.

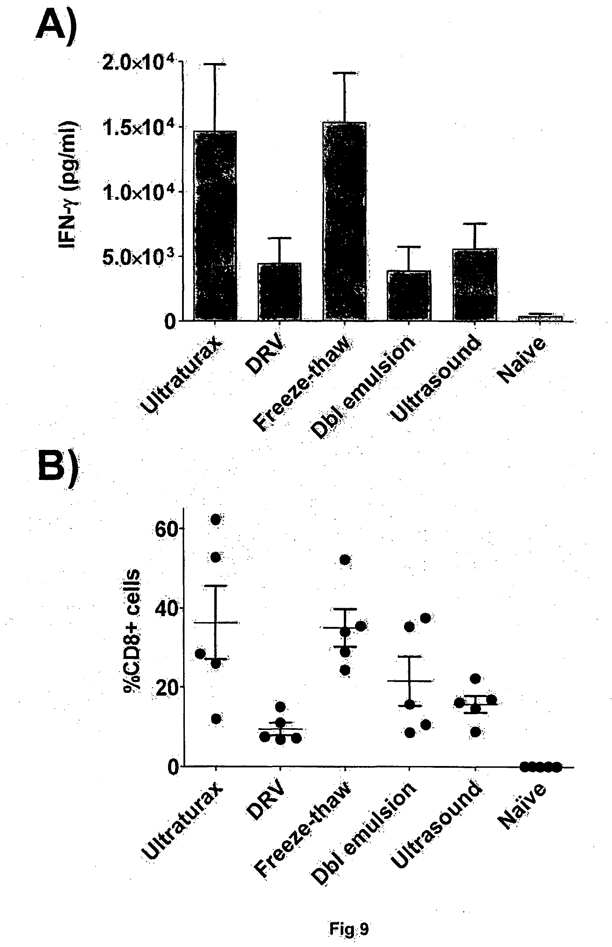

**Fig. 9** A) Release of IFN-gamma from blood lymphocytes isolated from C57BI/6j mice immunized with 5 µg of ovalbumin in CAF05 (DDA/TDB/poly (I:C)) prepared by high-shear mixing, DRV, freeze-thaw, double-emulsion and ultrasound methods. Splenocytes were isolated 3 weeks after the third immunization and restimulated in vitro with 5 µg/ml of ovalbumin. B) % SIINFEKL-positive CD8+ T-cells after three immunizations with 5 µg of ovalbumin in CAF05 (DDA/TDB/poly (I:C)) prepared by high-shear mixing, DRV, freeze-thaw, double-emulsion and ultrasound methods.

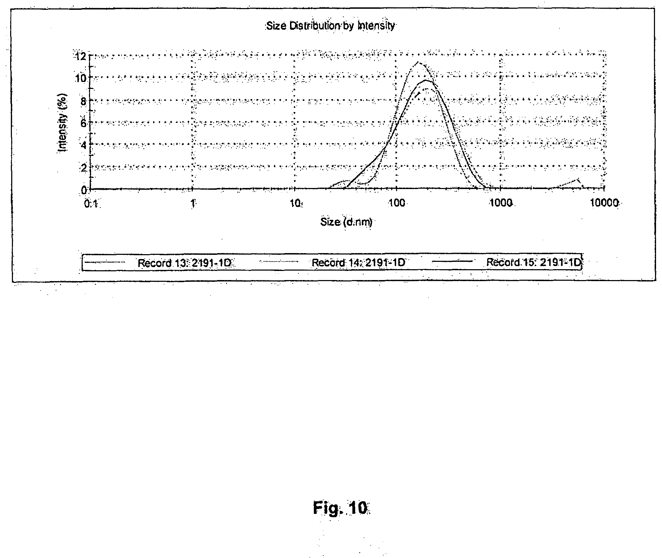

**Fig 10****.** Size distribution of H56-CAF01 vaccine manufactured using high shear mixing. Particle size distribution was 148,7±0,6 d.nm and measured using a Malvern Zetasizer-Nano from Malvern Instruments Ltd., UK.

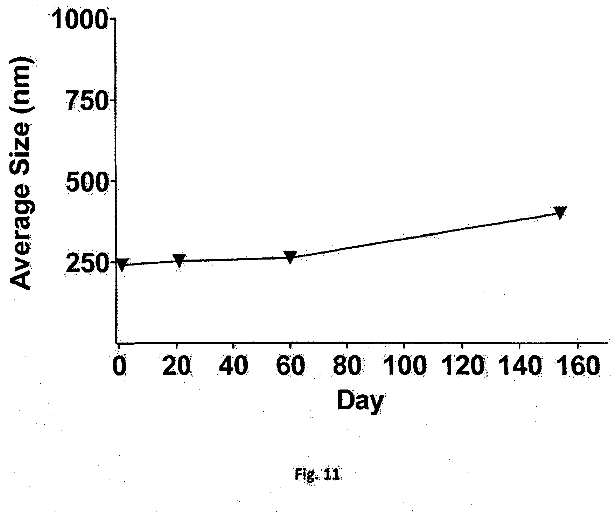

**Fig. 11** DLS stability of CAF05 (DDA/TDB/poly (I:C)). The average particle size (n=3) measured by dynamic light scattering over time. The CAF05 formulation is stable and within specifications for 160 days on storage at 2-8 °C.

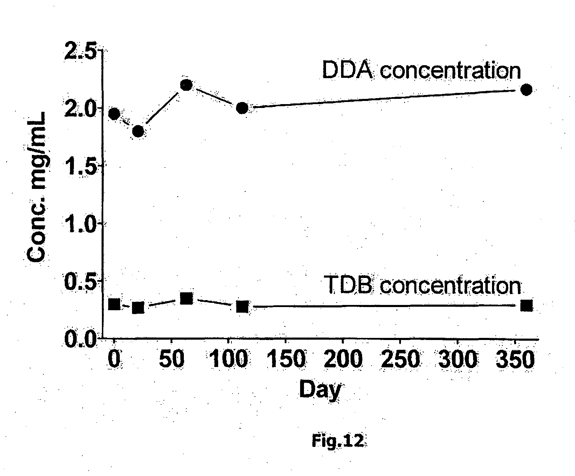

**Fig. 12** HPLC stability of CAF05 (DDA/TDB/poly (I:C))
   The DDA and TDB content measured in CAF05 over time. The content of the two polar lipids DDA and TDB of the CAF05 adjuvant samples is quantified using reversed phase HPLC and detection by Evaporate Light Scattering Detection (ELSD). The DDA and TDB contents in the CAF05 adjuvant are unchanged, stable and within specifications for 360 days, showing that CAF05 is stable when manufactured according to the disclosed invention.

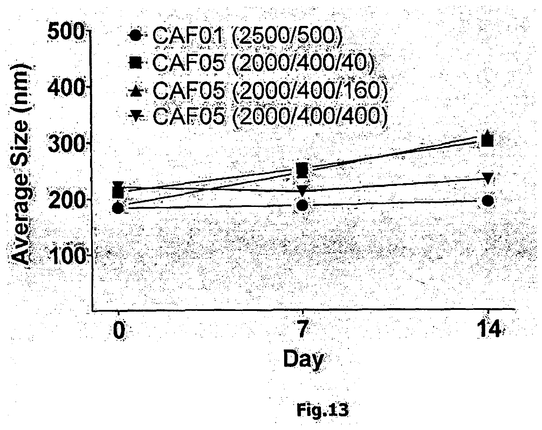

**Fig. 13** Particle size of stable CAF05 formulations of increasing Poly IC content.
   The formulations of CAF05 (DDA/TDB/poly (I:C)) with different DDA/TDB : poly (I:C) ratio from 5/1 : 0.1 to 5/1 : 1 are all within 157-220 nm in diameter at time zero. It is observed that the increase in particle size over time is more pronounced for the higher poly (I.C) containing formulations, though all being stable.

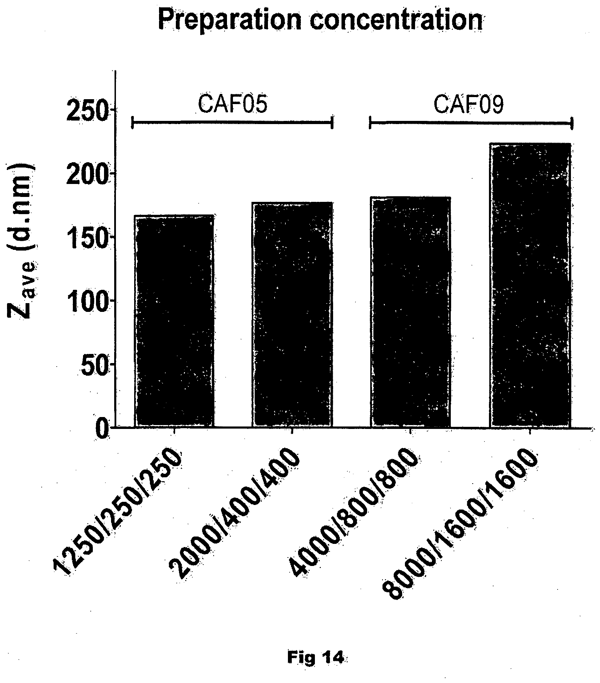

**Fig. 14** Particle size versus concentration.
   The formulations of CAF05 (DDA/TDB/poly (I:C)) and CAF09 (DDA/MMG/poly (I:C)) are all within 165-225 nm in diameter at time zero for all concentrated bulk productions.

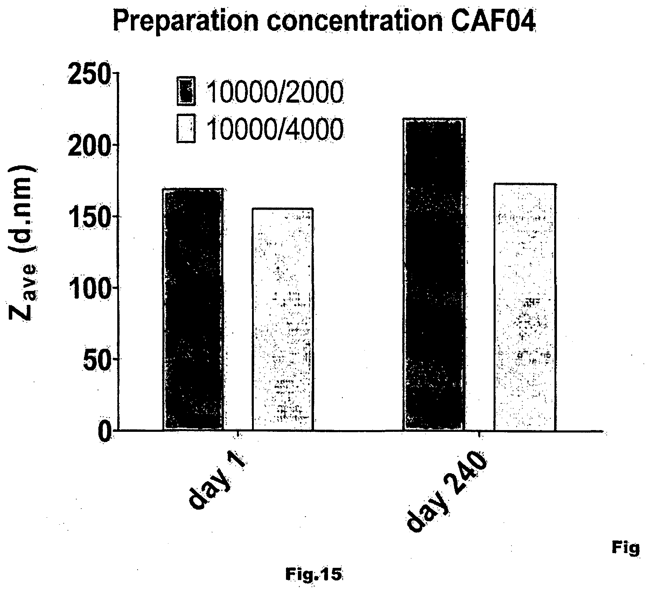

**Fig 15** DLS stability of CAF04 (DDA/MMG). MMG here is the analog 3-hydroxy-2-tetradecyl-octadecanoic acid-2,3-dihydroxypropyl ester. The average particle size (n=3) measured by dynamic light scattering over time. The CAF04 formulation is stable and within specifications for 240 days on storage at 2-8 □C.

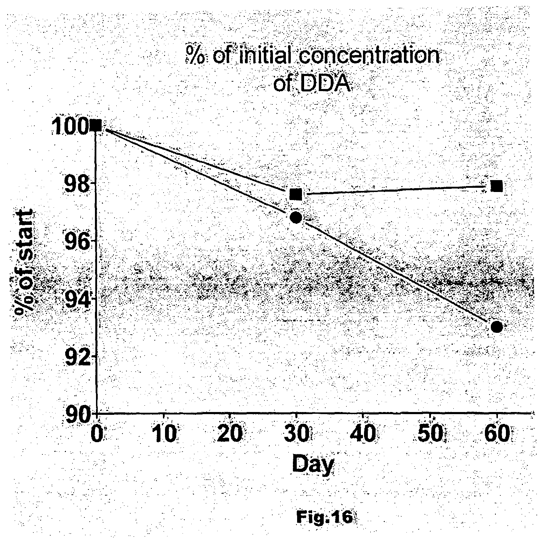

**Fig. 16** HPLC stability of CAF04 (DDA/MMG)
   The DDA content measured in CAF04 over time. ● DDA/MMG analog 10.000/4.000; ■ DDA/MMG analog 10.000/2.000. The content of the polar lipid DDA of the CAF04 adjuvant samples is quantified using reversed phase HPLC and detection by Evaporate Light Scattering Detection (ELSD). The DDA content in the CAF04 adjuvant is unchanged, stable and within specifications for 60 days, showing that CAF04 is stable when manufactured according to the disclosed invention.

### Examples

### Example 1: Determination of the melting temperatures of DDA, TDB and the lipid powder mixture using differential scanning calometry (DSC)

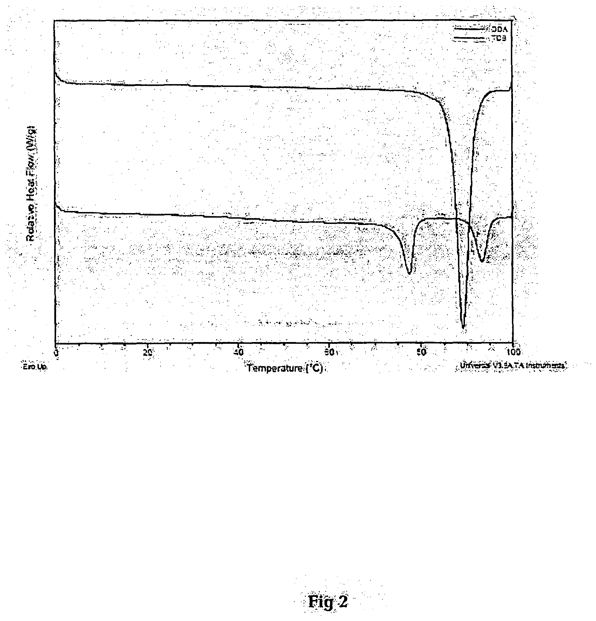

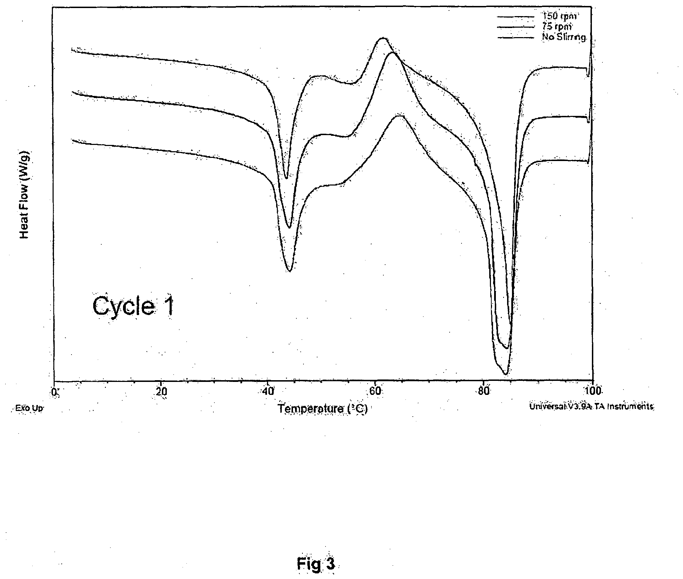

DSC thermographs of the starting material and resulting powder following the super critical CO₂ fluid method are presented in fig. 2 and fig. 3. The thermographs were obtained using microcalorimeter from TA instruments. Data acquisition and analysis was performed using TA instruments universal software v.3.9A.
In fig. 2 the phase transition of DDA showed a narrow peak with a top point of 89°C. The phase transition of TDB showed two peaks with top points at 77 and 93°C respectively.
The thermographs presented in fig. 3 show that there is no effect of stirring during the super critical method (RESS). The powder prepared using the super critical fluid method shows an intrinsic similarity to the powder prepared by the lipid film method in which organic solvents/co-solvents are used.
The conditions for the super critical fluid method for the obtained graphs in fig. 3 were 75°C and 140 bar.

### Example 2: Producing DDA/TDB lipid powder without the use of ethanol using the supercritical carbon dioxide process.

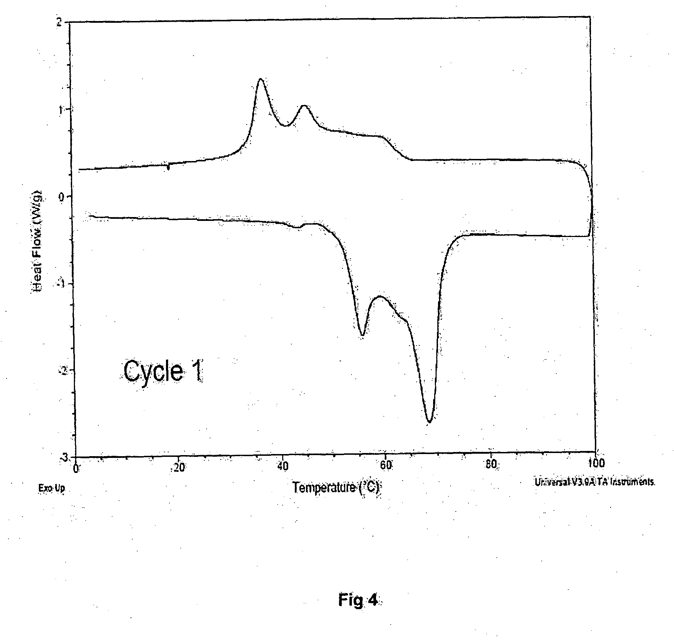

DSC thermographs of DDA/TDB lipid powders (DDA:TDB ratio 5:1 w/w) are shown in fig 4. The lipid components are loaded onto a standard autoclave, sealed and heated to the operating conditions which for the super critical fluid method for the obtained graphs in fig. 4 were 75°C and 140 bar. No co-solvent was added. The liquefied suspension was stirred, whereafter the sample was cooled to room temperature before venting. The obtainable lipid powder cake could easily be ground to fine powders.

### Example 3: Producing stable CAF01 (DDA/TDB) liposomes by high shear mixing

Liposome powder is weighed according to the desired final lipid content. The desired buffer is added e.g. 10 mM Tris pH 7.4 according to the weighed lipid content and desired final content. The buffer and lipid powder is heated to a temperature above the phase transition temperature Tₘ at the same time as high shear mixing at low speed is applied. Higher speed levels can be applied, however the risk of undesirable foaming increases with speed. When the temperature Tₘ is reached the high shear mixing at high rotational speed is started. The mixture is stirred at high rotational speed until a desired size distribution is reached. The liposome mixture is cooled to room temperature before being refrigerated.

### Example 4: Producing stable CAF05 (DDA/TDB/poly (I:C)) liposomes by high shear mixing

CAF05 is composed of the three components DDA, TDB and poly(I:C). First, DDA/TDB (CAF01) hydrated using high shear mixing is cooled to 60°C. Poly(I:C) solution at 60°C is added dropwise under the liquid surface to the liposome solution while high shear mixing is maintained. When the addition is completed the high shear mixing is continued for approximately 5 min. The CAF05 solution is cooled to room temperature before refrigeration. Stability data are shown in fig. 11 (particle size distribution) and fig. 12 (HPLC for DDA and TDB). By applying the manufacturing method disclosed in this invention, it is possible to manufacture CAF05 formulations of high lipid and poly (I:C) content. Examples are shown in fig 14 for CAF05 formulation of up to DDA/TDB/poly (I:C) contents of 8000 ug, 1600 ug and 1600 ug per mL, respectively.

### Example 5: Determination of the melting temperatures of DDA and CAF01 (DDA/TDB) liposomes produced using high shear mixing

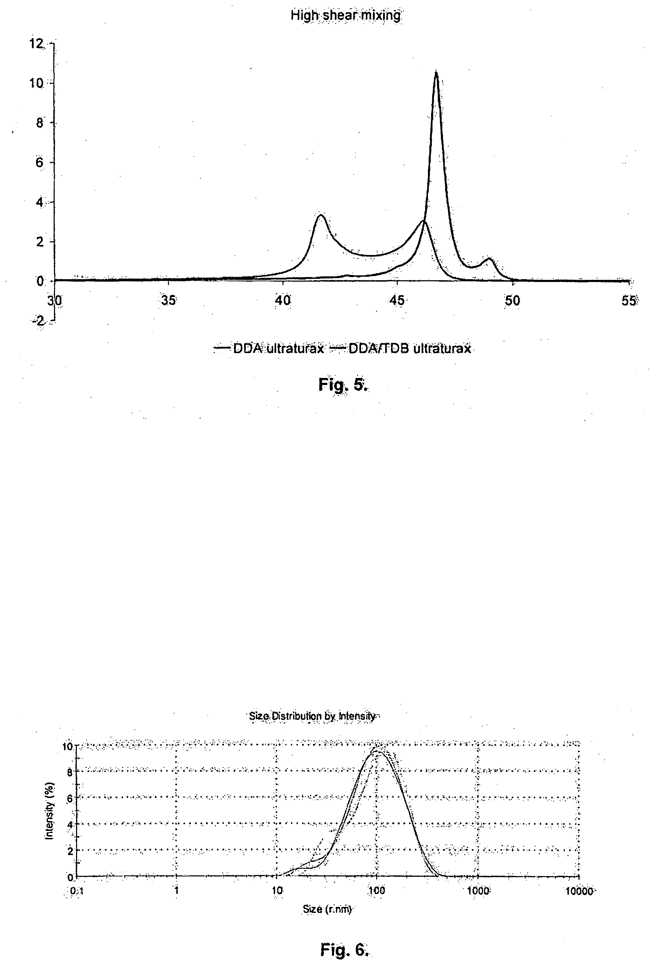

DSC thermographs of the liposomes are presented in fig. 5. The phase transition of DDA showed a narrow peak with a top point of 46,7°C. The phase transition of DDA/TDB was a broad peak from 39 to 47.5°C with two top points at 41.6 and 46,1°C respectively.
The calorimetric experiments were performed using a VP_DSC Microcalorimeter (Microcal, Northhampton, MA). A scan rate of 30 °C/h from 30 to 55 °C was used. The operating pressure was 35 psi. The total lipid concentration was 2750 µg/mL. Data acquisition and analysis was performed using Microcal's Origin 7 software. The phase transition temperatures are defined as the temperature at the peak maximum.

### Example 6: Size distribution of the liposomes

The average sizes of the liposomes were measured with dynamic light scattering on a Malvern Zetasizer-Nano series from Malvern Instruments Ltd, UK. The data were obtained at 25°C and the measurements were collected three times. Malvern Zetasizer PCS v.6.20 software was used to gather and calculate data. The size distribution of CAF01 (DDA/TDB) liposomes rehydrated using high shear mixing is presented in fig. 6. The average hydrodynamic diameter of the CAF01 liposomes presented in fig. 6 was 163,2±0,493 nm. The polydispersity index for the measurements was 0,258±0,008.

### Example 7: Stability of CAF01 (DDA/TDB) liposomes

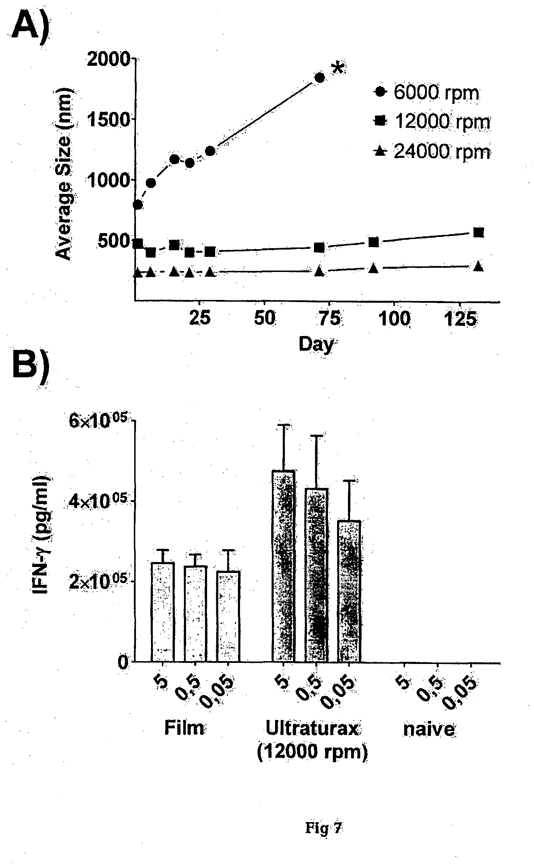

The stability of particles of CAF01 (DDA/TDB) prepared by the high shear mixing method at 6000, 12000 and 24000 rpm was determined by measuring the particle size over time by dynamic light scattering measurements using a Malvern ZetaSizer nanoZS (Malvern Instruments Ltd. UK). Formulations of CAF01 (DDA/TDB) were dispersed in 10mM Tris buffer with pH 7.4 on day 0. Measurements were done on day 1, 6, 15, 21, 29, 71, 92, and 132 after preparation (Figure 7A). Comparison of the particle size stability over time shows that stable liposomes are obtained by high shear mixing at 12000 rpm and 24000 rpm. In contrast, preparation at 6000 rpm resulted in particle size increase and visible aggregation was observed 71 days of storage at 4 °C and after day 92 no further particle size measurements were possible due to aggregation.

### Example 8: H56-CAF01 TB vaccine

The vaccine candidate H56-CAF01 comprises CAF01 (DDA/TDB) liposomes complexed with the H56 recombinant subunit protein (ESAT6-Ag85B-Rv2660). DDA/TDB hydrated using high shear mixing is cooled to room temperature, while high shear mixing is maintained. An aqueous solution of the H56 protein antigen is added slowly while high-shear mixing is maintained for approximately 5 min. In fig 10 is shown that applying this procedure, a vaccine is obtainable with no observed aggregation detected. The average hydrodynamic diameter of the CAF01 (DDA/TDB) liposomes presented in fig. 10 was 148,7±0,600 nm. The polydispersity index for the measurements was 0,251±0,007.

### Example 9: Stability of CAF05 (DDA/TDB/poly(I:C)) liposomes

The stability of particles of CAF05 (DDA/TDB/poly (I:C)) liposomes prepared by the high shear mixing method with varying contents of poly (I:C) was determined by measuring the particle size over time by dynamic light scattering measurements using a Malvern Zetasizer nano (Malvern Instruments Ltd. UK). Formulations of CAF05 (DDA/TDB/poly (I:C)) were prepared as described in example 4 for different ratios of DDA/TDB : poly (I:C) in Tris buffer with pH 7.4 on day 0. Measurements for particle size distribution were performed on days 0, 7 and 14 after preparation (Fig. 13). Comparison of the particle size stability over time shows that stable CAF05 liposomes are obtained by high shear mixing according to the disclosed invention.

### Example 10: Comparison of immunogenicity of liposomes prepared by high-shear mixing and lipid hydration with whirl mixing.

Mice were immunized subcutaneously (s.c.) at the base of the tails three times with a two week interval between each immunization. The vaccines (0.2 ml/mice) consisted of 2 µg of the fusion protein Ag85B-ESAT-6, emulsified in 300 µg CAF01 (DDA/TDB). Three weeks after the last immunization, blood lymphocytes were purified and the level of IFN-γ release measured after restimulation *in vitro* with 0.05, 0.5 and 5 µg of Ag85B-ESAT-6. High-shear mixing at 12000 rpm compared to the immune response generated with the whirl-mixing method, resulted in higher (although not significantly) IFN-γ responses (Figure 7B). Furthermore the antigen avidity was maintained as observed with the unchanged recall responses after re-stimulation with the low antigen concentration (Figure 7B).

### Example 11: Comparison of immunogenicity of CAF01 liposomes prepared by high-shear mixing at 6000, 12000 and 24000 rpm.

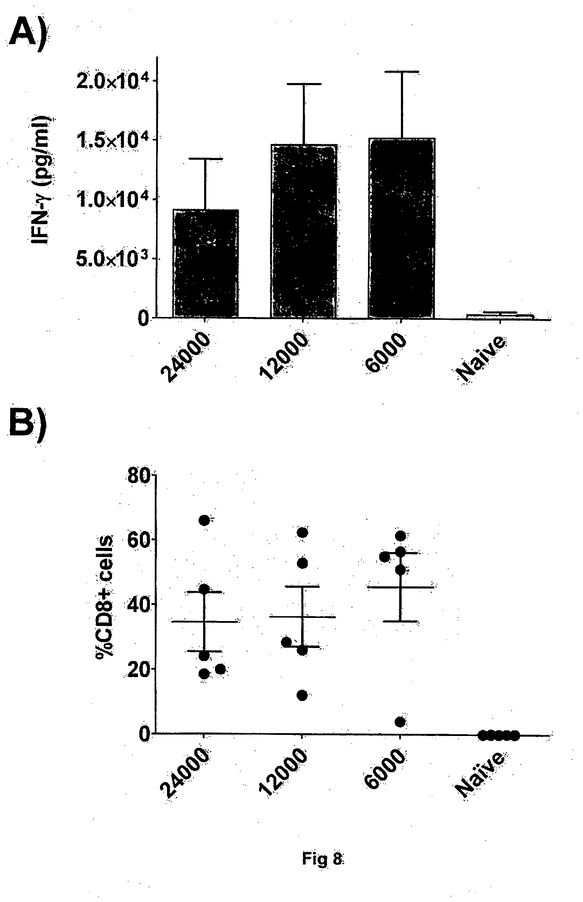

Mice were immunized subcutaneously (s.c.) at the base of the tails three times with a two week interval between each immunization. The vaccines (0.2 ml/mice) consisted of 5 µg of ovalbumin, emulsified in 300 µg CAF01 prepared by the high shear mixing method at 6000, 12000 and 24000 rpm. Three weeks after the last immunization, splenocytes were purified from individual mice and the level of IFN-γ release measured after re-stimulation *in vitro* with 5 µg of ovalbumin (Figure 8A). No significant changes was observed between the different formulations although the 24000 rpm method resulted in a slightly lower response.
In addition the amount of CD8+ cells was investigated by FACS: Isolated splenocytes were stimulated for 1h with 5 µg/ml OVA or 5 µg/ml of the CD8 peptide epitope of OVA in the presence of 1 µg/ml anti-CD28 and anti-CD49d. The cells were subsequently incubated for 5-6 h at 37 °C after addition of 10 µg/ml Brefeldin A and 0.7 µl/ml Monensin/Golgi-stop. Following overnight storage at 4 °C, the cells were washed in FACS buffer, and subsequently stained for surface markers for 30 min at 4 °C with mAb using 1:200 dilution of anti-CD8-PerCP-Cy5.5 in FACS buffer.
The stained cells were examined by flow cytometry immediately, using a FACScan flow cytometer (BD). No significant differences in the % of CD+ cells were obtained among the three formulations (Figure 8B).

### Example 12: Comparison of immunogenicity of CAF01 liposomes prepared by high-shear mixing, DRV, freeze-thaw, double-emulsion and ultrasound methods.

Mice were immunized subcutaneously (s.c.) at the base of the tails three times with a two week interval between each immunization. The vaccines (0.2 ml/mice) consisted of 5 µg of ovalbumin, emulsified in 300 µg CAF01 (DDA/TDB) adjuvant prepared by high-shear mixing (according to the invention), or DRV, freeze-thaw, double-emulsion and ultrasound methods (reference examples). Three weeks after the last immunization, were purified from individual mice and the level of IFN-γ release measured after re-stimulation *in vitro* with 5 µg of ovalbumin (Figure 9A). No significant changes were observed between the different formulations although the high-shear mixing and freeze-thaw methods resulted in higher responses than the rest.
In addition the amount of CD8+ cells were investigated by FACS: Isolated splenocytes were stimulated for 1h with 5 µg/ml OVA or 5 µg/ml of the CD8 peptide epitope of OVA in the presence of 1 µg/ml anti-CD28 and anti-CD49d. The cells were subsequently incubated for 5-6 h at 37 °C after addition of 10 µg/ml Brefeldin A and 0.7 µl/ml Monensin/Golgi-stop. Following overnight storage at 4 °C, the cells were washed in FACS buffer, and subsequently stained for surface markers for 30 min at 4 °C with mAb using 1:200 dilution of anti-CD8-PerCP-Cy5.5 in FACS buffer. The stained cells were examined by flow cytometry immediately, using a FACScan flow cytometer (BD). In line with the above mentioned results high-shear mixing and freeze-thaw based formulations induced significantly higher CD8 responses than those obtained by the DRV method but not the double-emulsion and ultrasound methods (Figure 9B).

### Example 13: Producing stable CAF09 (DDA/MMG/poly (I:C)) liposomes by high shear mixing

CAF09 is composed of the three components DDA, a synthetic MMG analog (3-hydroxy-2-tetradecyl-octadecanoic acid-2,3-dihydroxypropyl ester) and poly(I:C). First, DDA/MMG (CAF04) hydrated using high shear mixing is cooled to 60°C. Poly(I:C) solution at 60°C is added dropwise under the liquid surface to the liposome solution, while high shear mixing is maintained. When the addition is completed the high shear mixing is continued for approximately 5 min. The CAF09 solution is cooled to room temperature before refrigeration. Data for particle size distribution of CAF09 (165-225 nm) manufactured according to the disclosed invention is shown in fig.14.

### Example 14: Various vaccine formulations with different liposome adjuvants produced by high shear mixing

Stable formulations of CAF01, CAF04, CAF05 and CAF09 liposomes produced with high shear mixing have been prepared with antigens from various disease targets like tuberculosis, HIV, influenza A and chlamydia (table 1).

**Table 1: Vaccine formulations.**

| Adjuvans | Antigen | Disease target |
|---|---|---|
| CAF01 | H1 | Tuberculosis |
| | H56 | Tuberculosis |
| | TB10.3 protein | Tuberculosis |
| | TB10.3-P1 | Tuberculosis |
| | Gag p24 | HIV |
| | rMOMP | Chlamydia |
| | CtH1 | Chlamydia |
| | Influenza split | Influenza |
| CAF05 | TB10.3 protein | Tuberculosis |
| | H56 | Tuberculosis |
| | Gag p24 | HIV |
| | H1 | Tuberculosis |
| | H4 | Tuberculosis |
| | TB10.4 | Tuberculosis |
| | Influenza split | Influenza |
| | CtH1 | Chlamydia |
| CAF04 | H56 | Tuberculosis |
| | rMOMP | Chlamydia |
| CAF09 | H56 | Tuberculosis |
| | TB10.3 | Tuberculosis |
| | OVA | Model antigen |
| | Influenza split | Influenza |

### References

Andersen CA, Rosenkrands I, Olsen AW, Nordly P, Christensen D, Lang R, Kirschning C, Gomes JM, Bhowruth V, Minnikin DE, Besra GS, Follmann F, Andersen P, Agger EM. Novel generation mycobacterial adjuvant based on liposome-encapsulated monomycoloyl glycerol from Mycobacterium bovis bacillus Calmette-Guerin. J Immunol. 183(4), 2294-302 (2009).
Andersen CS, Agger EM, Rosenkrands I, Gomes JM, Bhowruth V, Gibson KJ, Petersen RV, Minnikin DE, Besra GS, Andersen P. A simple mycobacterial monomycolated glycerol lipid has potent immunostimulatory activity. J Immunol. 182(1), 424-32 (2009).
Badens, E., Magnan, C., Charbit, G. "Microparticles of Soy Lecithin formed by supercritical processes" Biotech. Bioeng. (72)2 194-204 (2001).
Bangham AD, Standish MM, Watkins JC. Diffusion of univalent ions across the lamellae of swollen phospholipids. J Mol Biol. 13(1), 238-52 (1965).
Cape, S.P., Villa, J.A., Huang, E.T.S., Yang, T-H., Carpenter, J.F., Sievers, R.E. "Preparation of active proteins, vaccines and pharmaceuticals as fine powders using supercritical or near-supercritical fluids" Pharm. Res. (25) 1967-1990 (2008).
Christensen D, Korsholm KS, Rosenkrands I, Lindenstrom T, Andersen P, Agger EM. Cationic liposomes as vaccine adjuvants. Expert review of vaccines. 6(5), 785-96 (2007).
Frederiksen, L., Anton, K., Hoogevest, P., Keller, H.R., Leuenberger, H. "Preparation of liposomes encapsulating water-soluble compounds using supercritical carbon dioxide" J. Pharm. Sci. (86) 921-928 (1997).
Gregoriadis G, Davis D, Davies A. Liposomes as immunological adjuvants: antigen incorporation studies. Vaccine. 5(2), 145-51 (1987).
Gregoriadis G, Garcon N, da Silva H, Sternberg B. Coupling of ligands to liposomes independently of solute entrapment: observations on the formed vesicles. Biochim Biophys Acta. 1147(2), 185-93 (1993).
Gregoriadis, G., McCormack, B., Obrenovic, M., Saffie, R., Zadi, B., Perrie, Y. "Vaccine entrapment in liposomes" Methods (19) 156-162 (1999).
Henriksen-Lacey M, Bramwell VW, Christensen D, Agger EM, Andersen P, Perrie Y. Liposomes based on dimethyldioctadecylammonium promote a depot effect and enhance immunogenicity of soluble antigen. J Control Release. 142(2), 180-6 (2010).
Justo, O.R., Moraes, A.M. "Economical feasibility evaluation of an ethanol injection liposome production plant" Chem. Eng. Technol. (33), 15-20 (2010).
Kirby, C., Gregoriadis, G. "Dehydration-rehydration vesicles (DRV): A new method for high yield drug entrapment in liposomes" Biotechnology (2) 979-984 (1984).
Mehnert, W., Mäder, K. "Solid lipid nanoparticles production, characterization and applications" Adv. Drug Deliv. Rev. (47) 165-196 (2001).
Nordly P, Korsholm KS, Pedersen EA, Khilji TS, Franzyk H, Jorgensen L, Nielsen HM, Agger EM, Foged C. Incorporation of a synthetic mycobacterial monomycoloyl glycerol analogue stabilizes dimethyldioctadecylammonium liposomes and potentiates their adjuvant effect in vivo. Eur J Pharm Biopharm. 77(1), 89-98 (2011).
Nordly P, Rose F, Christensen D, Nielsen HM, Andersen P, Agger EM, Foged C. Immunity by formulation design: induction of high CD8+ T-cell responses by poly(I:C) incorporated into the CAF01 adjuvant via a double emulsion method. J Control Release. 150(3), 307-17 (2011).
Pasquali, I., Bettini, R., Giordano, F. "Supercritical fluid te3chnologies: an innovative approach for manipulating the solid-state of pharmaceuticals" Adv. Drug Deliv. Rev. (60) 399-410 (2008).
Pick U. Liposomes with a large trapping capacity prepared by freezing and thawing of sonicated phospholipid mixtures. Archives of biochemistry and biophysics. 212(1), 186-94 (1981).
Ribeiro AM, Chaimovich H. Preparation and characterization of large dioctadecyldimethylammonium chloride liposomes and comparison with small sonicated vesicles. Biochim Biophys Acta. 733(1), 172-9 (1983).
Szoka F, Jr., Papahadjopoulos D. Procedure for preparation of liposomes with large internal aqueous space and high capture by reverse-phase evaporation. Proceedings of the National Academy of Sciences of the United States of America. 75(9), 4194-8 (1978).
Wagner, A., Platzgummer, M., Kreismayr, G., Quendler, H., Stiegler, G., Ferko, B., Vecera, G., Vorauer-Uhl, K., Prof, H.K. "GMP production of liposomes - a new industrial approach" J. Liposomes Res (16) 311-319 (2006).
Zadi, B., Gregoriadis,. G. "A novel method for high-yield entrapment of solutes into small liposomes" J. Liposomes Res. (10) 73-80 (2000).

## Claims

1. A method of producing liposomes free of organic solvents, the method being without the use of organic solvents using high shear mixing for hydrating a lipid powder in an aqueous solution, wherein the lipid powder is a homogenous mixture where one lipid component is a quaternary ammonium compound having the general formula NR¹R²R³R⁴-X wherein R¹ and R² independently each is a short chain alkyl group containing from 1 to 3 carbon atoms, R³ is independently hydrogen or a methyl or an alkyl group containing from 12 to 20 carbon atoms, preferably 14 to 18 carbon atoms, R4 is independently a hydrocarbon group containing from 12 to 20 carbon atoms, preferably from 14 to 18 carbon atoms, and X is a nontoxic pharmaceutically acceptable anion, and one component is a neutral lipid or a glycolipid, wherein said lipid powder is obtained without the use of organic solvents from a supercritical fluid or by cooling of a melt, and wherein said high shear mixing is based on the rotor-stator principle.

2. A method of producing liposomes according to claim 1, where high shear mixing is based on the rotor-stator principle where the rotational speed of the rotor is at least 3,400 rpm.

3. A method of producing liposomes according to claim 1 or 2, where the lipid powder is produced by a supercritical fluid process.

4. A method of producing liposomes according to any of claims 1-3, where the quaternary ammonium compound is dimethyldioctadecylammonium (DDA), or dimethyldioctadecenylammonium (DODA), or 1,2-dioleoyl-3-trimethylammonium propane (DOTAP), 1,2-dimyristoyl-3-trimethylammonium-propane, 1,2-dipalmitoyl-3-trimethylammonium-propane, 1,2-distearoyl-3-trimethylammonium-propane and dioleoyl-3-dimethylammonium propane (DODAP), N-[1-(2,3-dioleyloxy)propyl]-N,N,N-trimethylammonium (DOTMA).

5. A method of producing liposomes according to any of claims 1-4, where the glycolipid is alpha,alpha'-trehalose 6,6'- dibehenate (TDB) or synthetic monomycolyl glycerol (MMG).

6. A method of producing liposomes according to claim 5, where the synthetic MMG is 3-hydroxy-2-tetradecyl-octadecanoic acid-2,3-dihydroxypropyl ester.

7. A method of producing liposomes according to any of claims 1-6, where the liposomes additionally comprise an immunopotentiator.

8. A method of producing liposomes according to claim 7, where the immunepotentiator is chosen from the C-type lectin receptors, nucleotide-binding oligomerization domain (NOD) proteins and the toll-like receptor (TLR) family, such as cord factor (TDM) or the synthetic analogue TDB, syntetic monomycolyl glycerol (MMG), flagellin, lipopolysaccharide, peptidoglycans or cytosine:phosphate:guanine (CpG) oligodeoxynucleotides and polyinosinic acid:polycytidylic acid (poly(I:C)).

9. A method of producing liposomes according to any of claims 1-8, where the liposomes are complexed with macromolecules such as oligonucleotides, peptides, proteins, carbohydrates and/or lipids.

10. A liposome product produced according to any preceding claim.

11. A liposome product according to claim 10, where the liposome comprises DDA and TDB or synthetic MMG or 3-hydroxy-2-tetradecyl-octadecanoic acid-2,3-dihydroxypropyl ester.

12. A liposome product according to claim 10 or 11, where the liposome further comprises poly(I:C).

13. A liposome product according to any of claims 10-12 comprising an antigen.

14. A liposome product according to claim 13, where the antigen is ESAT6-Ag85B or ESAT6-Ag85B-Rv2660.

15. A liposome product according to any of claims 10-14 for use as an adjuvant, a vaccine, a delivery system, a pharmaceutical or a cosmeceutical.

## Patentansprüche

1. Verfahren zum Herstellen von Liposomen, die frei von organischen Lösungsmitteln sind, wobei das Verfahren ohne die Verwendung organischer Lösungsmittel unter Verwendung eines Hochschermischens durchgeführt wird, um ein Lipidpulver in einer wässrigen Lösung zu hydratisieren, wobei das Lipidpulver ein homogenes Gemisch ist, bei dem eine Lipidkomponente eine quartäre Ammoniumkomponente ist, welche die allgemeine Formel NR¹R²R³R⁴-X aufweist, wobei R¹ und R² jeweils unabhängig eine kurzkettige Alkylgruppe sind, die 1 bis 3 Kohlenstoffatome enthält, R³ unabhängig Wasserstoff oder eine Methyl- oder eine Alkylgruppe ist, die 12 bis 20 Kohlenstoffatome, vorzugsweise 14 bis 18 Kohlenstoffatome, enthält, R4 unabhängig eine Kohlenwasserstoffgruppe ist, die 12 bis 20 Kohlenstoffatome, vorzugszweie 14 bis 18 Kohlenstoffatome, enthält und X ein nicht toxisches pharmazeutisch unbedenkliches Anion ist, und eine Komponente ein neutrales Lipid oder ein Glycolipid ist, wobei das Lipidpulver ohne die Verwendung organischer Lösungsmittel aus einem überkritischen Fluid oder durch das Abkühlen einer Schmelze erhalten wird, und wobei das Hochschermischen auf dem Rotor-Stator-Prinzip basiert ist.

2. Verfahren zum Herstellen von Liposomen nach Anspruch 1, wobei das Hochschermischen auf dem Rotor-Stator-Prinzip basiert ist, wobei die Rotationsgeschwindigkeit des Rotors mindestens 3.400 U/min beträgt.

3. Verfahren zum Herstellen von Liposomen nach Anspruch 1 oder 2, wobei das Lipidpulver durch einen überkritischen Fluidprozess hergestellt wird.

4. Verfahren zum Herstellen von Liposomen nach einem der Ansprüche 1-3, wobei die quartäre Ammoniumverbindung Dimethyldioctadecylammonium (DDA) oder Dimethyldioctadecenylammonium (DODA) oder 1,2-Dioleoyl-3-trimethylammoniumpropan (DOTAP), 1,2-Dimyristoyl-3-trimethylammoniumpropan, 1,2-Dipalmitoyl-3-trimethylammoniumpropan, 1,2-Distearoyl-3-trimethylammoniumpropan und Dioleoyl-3-dimethylammoniumpropan (DODAP), N-[1-(2,3-Dioleyloxy)propyl]-N,N,N-trimethylammonium (DOTMA) ist.

5. Verfahren zum Herstellen von Liposomen nach einem der Ansprüche 1-4, wobei das Glycolipid alpha,alpha'-Trehalose-6,6'-dibehenat (TDB) oder synthetisches Monomycolylglycerol (MMG) ist.

6. Verfahren zum Herstellen von Liposomen nach Anspruch 5, wobei das synthetische MMG 3-Hydroxy-2-tetradecyloctadecansäure-2,3-dihydroxypropylester ist.

7. Verfahren zum Herstellen von Liposomen nach einem der Ansprüche 1-6, wobei die Liposome zusätzlich einen Immunpotentiator umfassen.

8. Verfahren zum Herstellen von Liposomen nach Anspruch 7, wobei der Immunpotentiator ausgewählt ist aus den C-Typ-Lectinrezeptoren, Proteinen der nukleotidbindenden Oligomerisationsdomäne (NOD) und der Familie des toll-ähnlichen Rezeptors (TLR), wie etwa Cord-Faktor (TDM) oder das synthetische Analogon TDB, synthetisches Monomycolylglycerol (MMG), Flagellin, Lipopolysaccharid, Peptidoglycane oder Cytosin:Phosphat:Guanin(CpG)-Oligodesoxynukleotide und Polyinosinsäure:Polycytidylsäure (Poly(I:C)).

9. Verfahren zum Herstellen von Liposomen nach einem der Ansprüche 1-8, wobei die Liposome mit Makromolekülen, wie etwa Oligonukleotiden, Peptiden, Proteinen, Kohlenhydraten und/oder Lipiden, komplexiert ist.

10. Liposomprodukt, hergestellt nach einem der vorhergehenden Ansprüche.

11. Liposomprodukt nach Anspruch 10, wobei das Liposom DDA und TDB oder synthetisches MMG oder 3-Hydroxy-2-tetradecyloctadecansäure-2,3-dihydroxypropylester umfasst.

12. Liposomprodukt nach Anspruch 10 oder 11, wobei das Liposom ferner Poly(I:C) umfasst.

13. Liposomprodukt nach einem der Ansprüche 10-12, umfassend ein Antigen.

14. Liposomprodukt nach Anspruch 13, wobei das Antigen ESAT6-Ag85B oder ESAT6-Ag85B-Rv2660 ist.

15. Liposomprodukt nach einem der Ansprüche 10-14 zur Verwendung als ein Adjuvans, ein Impfstoff, ein Abgabesystem, ein Pharmazeutikum oder ein Kosmetikum.

## Revendications

1. Procédé de production de liposomes exempts de solvants organiques, le procédé étant sans utilisation de solvants organiques en utilisant un mélange à cisaillement élevé pour hydrater une poudre lipidique en solution aqueuse, ladite poudre lipidique étant un mélange homogène où un composant lipidique est un composé ammonium quaternaire représenté par la formule générale NR¹R²R³R⁴-X dans laquelle R¹ et R² représentent chacun indépendamment un groupe alkyle à chaîne courte contenant de 1 à 3 atomes de carbone, R³ représentant indépendamment un atome d'hydrogène ou un groupe méthyle ou un groupe alkyle contenant de 12 à 20 atomes de carbone, de préférence 14 à 18 atomes de carbone, R⁴ représente indépendamment un groupe hydrocarboné contenant de 12 à 20 atomes de carbone, de préférence de 14 à 18 atomes de carbone, et X représente un anion non toxique pharmaceutiquement acceptable, et un composant est un lipide neutre ou un glycolipide, ladite poudre lipidique étant obtenue sans utiliser de solvants organiques à partir d'un fluide supercritique ou par refroidissement d'une matière en fusion, et ledit mélange à cisaillement élevé se basant sur le principe rotor-stator.

2. Procédé de production de liposomes selon la revendication 1, ledit mélange à cisaillement élevé se basant sur le principe rotor-stator où la vitesse de rotation du rotor vaut au moins 3400 rpm.

3. Procédé de production de liposomes selon la revendication 1 ou 2, où la poudre lipidique est produite par un procédé à fluide supercritique.

4. Procédé de production de liposomes selon l'une quelconque des revendications 1 à 3, où le composé ammonium quaternaire est le diméthyldioctadécylammonium (DDA), ou le diméthyldioctadécénylammonium (DODA), ou le 1,2-dioléoyl-3-triméthylammonium propane (DOTAP), le 1,2-dimyristoyl-3-triméthylammonium-propane, le 1,2-dipalmitoyl-3-triméthylammonium-propane, le 1,2-distéaroyl-3-triméthylammonium-propane et le dioléoyl-3-diméthylammonium-propane (DODAP), le N-[1-(2,3-dioléyloxy)propyl]-N,N,N-triméthylammonium (DOTMA).

5. Procédé de production de liposomes selon l'une quelconque des revendications 1 à 4, où le glycolipide est l'alpha,alpha'-tréhalose-6,6'-dibéhénate (TDB) ou le monomycolylglycérol (MMG) synthétique.

6. Procédé de production de liposomes selon la revendication 5, où le MMG synthétique est le 2,3-dihydroxypropylester d'acide 3-hydroxy-2-tétradécyl-octadécanoïque.

7. Procédé de production de liposomes selon l'une quelconque des revendications 1 à 6, où les liposomes comprennent en outre un immunostimulateur.

8. Procédé de production de liposomes selon la revendication 7, où l'immunostimulateur est choisi parmi les récepteurs de lectine de type C, les protéines à domaine d'oligomérisation se liant aux nucléotides (NOD) et la famille des récepteurs de type toll (TLR), tel que le tréhalose-6,6'-dimycolate (TDM) ou l'analogue synthétique (TDB), le monomycolylglycérol (MMG) synthétique, la flagelline, le lipopolysaccharide, les peptidoglycanes ou les oligodésoxynucléotides cytosine:phosphate:guanine (CpG) et l'acide polyinosinique:acide polycytidylique (poly(I:C)).

9. Procédé de production de liposomes selon l'une quelconque des revendications 1 à 8, où les liposomes sont complexés avec des macromolécules telles que des oligonucléotides, des peptides, des protéines, des glucides et/ou des lipides.

10. Produit liposomique produit selon l'une quelconque des revendications précédentes.

11. Produit liposomique selon la revendication 10, où le liposome comprend du DDA et du TDB ou du MMG synthétique ou du 2,3-dihydroxypropyl-ester d'acide 3-hydroxy-2-tétradécyl-octadécanoïque.

12. Produit liposomique selon la revendication 10 ou 11, où le liposome comprend en outre du poly(I:C).

13. Produit liposomique selon l'une quelconque des revendications 10 à 12 comprenant un antigène.

14. Produit liposomique selon la revendication 13, où l'antigène est ESAT6-Ag85B ou ESAT6-Ag85B-Rv2660.

15. Produit liposomique selon l'une quelconque des revendications 10 à 14 pour utilisation comme adjuvant, vaccin, système d'administration, produit pharmaceutique ou produit cosméceutique.
